# EUROPEAN PATENT SPECIFICATION

(11) **EP 1 809 609 B1**
(45) Date of publication and mention of the grant of the patent: **16.04.2008**
(21) Application number: 05800358.3
(22) Date of filing: 04.11.2005
(51) Int. Cl.: C07D 239/30, C07D 239/36

(54) **PROCESS FOR THE PRODUCTION OF PYRIMIDINE-5-CARBOXYLATES**
VERFAHREN ZUR HERSTELLUNG VON PYRIMIDIN-5-CARBOXYLATEN
PROCEDE DE PRODUCTION DE PYRIMIDINE-5-CARBOXYLATES

(30) Priority: 05.11.2004 EP 04026254
(43) Date of publication of application: 25.07.2007
(73) Proprietor: Lonza AG, 4052 Basel (CH)
(72) Inventor: DUCRY, Laurent, CH-3960 Sierre (CH); RITTINER, Bruno, CH-3902 Brig-Glis (CH)
(86) International application number: PCT/EP2005/011802
(87) International publication number: WO 2006/048297

(56) References cited:
- EP-A- 0 569 912
- PALANKI M S S ET AL: "INHIBITORS OF NF-KAPPABETA AND AP-1 GENE EXPRESSION: SAR STUDIES ONTHE PYRIMIDINE PORTION OF 2-CHLORO-4-TRIFLUOROMETHYLPYRIMIDINE-5- N-(3',5'-BIS(TRIFLUOROMETHYL)-PHENYL)CARBO XAMIDEL" JOURNAL OF MEDICINAL CHEMISTRY, AMERICAN CHEMICAL SOCIETY. WASHINGTON, US, vol. 43, no. 21, 2000, pages 3995-4004, XP000986229 ISSN: 0022-2623 cited in the application

## Description

The present invention relates to a process for the production of pyrimidine-5-carboxylates of formula 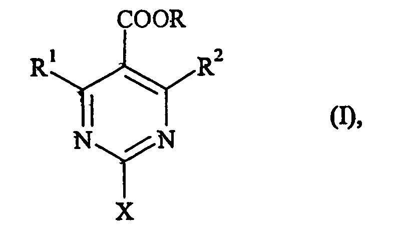 wherein R is C₁₋₄ alkyl, R¹ is C₁₋₄ alkyl, trifluoromethyl or optionally substituted phenyl, R² is hydrogen or C₁₋₄ alkyl and X is hydroxy, chlorine or bromine, or hydrates of said pyrimidine-5-carboxylates wherein X is hydroxy. It further relates to said hydrates as novel compounds.

Here and hereinbelow, C₁₋₄ alkyl is to be understood as meaning any linear or branched alkyl group having 1 to 4 carbon atoms, in particular methyl, ethyl, propyl, isopropyl, butyl, isobutyl, *sec-butyl* or *tert-butyl.* Phenyl groups may be substituted with any substituent that does not interfere with the reaction. In particular, a phenyl group may be substituted with one up to five substituents which may be the same or different and may be selected from C₁₋₄ alkyl, halogen, C₁₋₄ alkoxy and the like.

Compounds of formula I, in particular the compounds wherein R¹ is trifluoromethyl and R² is hydrogen, are valuable intermediates, e.g. for the production of fungicides (EP-A-0 569 912) and antiinflammatory compounds (U5-A-5 852 028).

A known synthesis of ethyl 2-hydroxy 4-(trifluoromethyl)pyrimidine-5-carboxylate (I, R = Et, R¹ = CF₃, R² = H, X = OH) comprises the reaction of ethyl 4,4,4-trifluoroacetoacetate and triethyl orthoformate to give ethyl 2-ethoxymethylene-4,4,4-trifluoroacetoacetate and the reaction of this ethoxymethylene compound with urea and subsequent hydrolysis of the intermediate ureidomethylene compound (M. S. S. Palanki et al., J. Med. Chem. 2000, 43, 3995-4004; US-A-5 852 028; cf. EP-A-0569 912). This three-step process has the disadvantages that drastic reaction conditions (acetic anhydride, 120-140 °C) are required in the first step and the overall process is tedious.

It is also possible to combine the reaction of ethyl trifluoroacetoacetate with ethyl orthoformate and urea in one step (E. D. Bergmann et al., J. Chem. Soc. Abstr. 1959, 3278-3285; Chem. Abstr. 1960, 54, 6736i-6738i) however, the yield is only moderate and the ureidomethylene compound has to be isolated before the cyclization reaction is carried out. It is also known that 2-hydroxypyrimidine-5-carboxylates can be converted into the 2-chloro compounds with phosphorus oxychloride (US-A-5 852 028). This reaction has the drawback that a large amount of phosphate waste is produced.

It was an object of the present invention to provide a simple alternative process for the preparation of pyrimidine-5-carboxylates of formula I that affords high yields and is appropriate for the production on an industrial scale.

It has been found that it is possible to conduct the synthesis of the 2-hydroxypyrimidine-5-carboxylates in a one-pot reaction under relatively moderate conditions without isolating any intermediate.
According to the invention, pyrimidine-5-carboxylates of formula 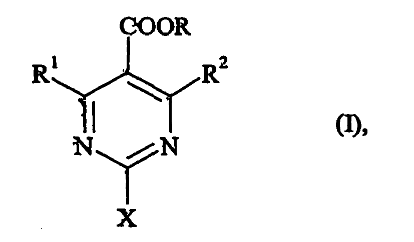 wherein R is C₁₋₄ alkyl, R¹ is C₁₋₄ alkyl or trifluoromethyl, R² is hydrogen or C₁₋₄ alkyl and X is hydroxy, chlorine or bromine, are prepared by
(i) reacting a 3-oxoalkanoate of formula 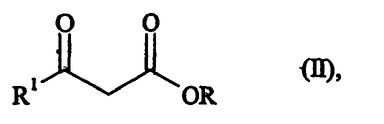 wherein R and R¹ are as defined above, with urea and an orthoester of formula

   R²C(OR)₃ (III),

   wherein R and R² are as defined above, to yield a 2-acyl-3-ureidoacrylate of formula 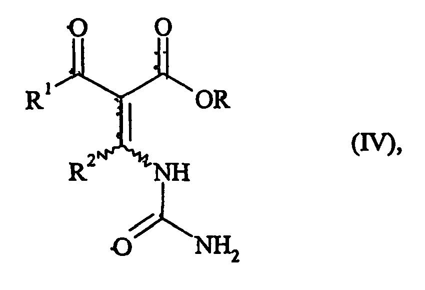 and
(ii) reacting said 2-acyl-3-ureidoacrylate (IV) to give a 2-hydroxypyrimidine-5-carboxylate of formula 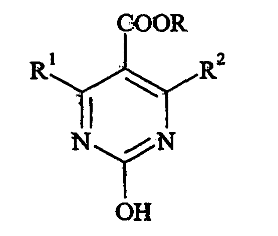 (I), X = OH
   wherein R is as defined above, and, optionally,
(iii) converting said 2-hydroxypyrimidine-5-carboxylate into a corresponding chloro or bromo compound (I, X = Cl, Br), whereby steps (i) anti (ii) are conducted in a one-pot reaction without isolating any intermediate.

It has been found that, depending on the work-up conditions and the nature of the substituents R¹ and R², the 2-hydroxypyrimidine-5-carboxylates may form hydrates of formula 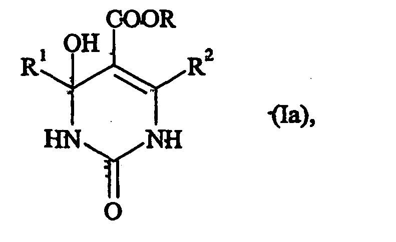 wherein R, R¹ and R² are as defined above, or tautomers thereof

The cyclization step (ii) may be conducted in the presence of a strong base, preferably an alkali alkoxide of formula

M-OR (V),

wherein M is an alkali metal and R is as defined above.

It has, however, been found that the presence of a base in the cyclization step(ii) is not mandatory and it is possible to conduct both reaction steps (i) and (ii) not only in a one-pot reaction, but also in the same process step by simply heating a mixture of the starting materials (II), (III) and urea without adding a base for a time sufficient to allow both reaction steps to proceed.

In a preferred embodiment, R in the 3-oxoalkanoate (II), the orthoester (III) and, if present, the alkoxide (V), as well as in the intermediate (IV) and the product (I), is ethyl.

In another preferred embodiment, R¹ in the 3-oxoalkanoate (H) is trifluoromethyl, i.e., the 3-oxoalkanoate is a trifluoroacetoacetate, thus affording a 4-trifluoromethylpyrimidine-5-carboxylate (I).

Preferably, R² in the orthoester (III) and, consequently, the 2-acyl-3-ureidoacrylate (IV) and the pyrimidine-5-carboxylate (1) is hydrogen, i.e., the orthoester is an orthoformate.

As alkali metal M in the alkali alkoxide (V), if present, any alkali metal, i.e., lithium, sodium, potassium, rubidium or caesium, may be employed. Preferably, the alkali metal is sodium.

The conversion of the 2-hydroxypyrimidine-5-carboxylates into the 2-chloro- or 2-bromopyrimidine-5-carboxylates can be carried out using methods for the conversion of 2-hydroxypyrimidines into 2-halopyrimidines which are known in the art.

As pyrimidine-5-carboxylates, the compounds wherein X is chlorine are pretended. The conversion of the 2-hydroxypyrimidine-5-carboxylates into the 2-chloropyrimidine-5-carboxylates is preferably carried out using phosphorus oxychloride or thionyl chloride, thionyl chloride in the presence of *N,N-*dimethylformamide being particularly preferred.

Reaction steps (i) and (ii) of the process according to the invention may be carried out in any inert solvent having a boiling point at or above the intended reaction temperature, such as aromatic hydrocarbons like toluene or ethers like tetrahydrofuran. It is even possible to use no solvent at all, in particular since three moles of alcohol (ROH) are formed as byproduct in reaction step (i).

In a preferred embodiment, reaction steps (i) and (ii) are carried out using an alcohol of formula R-OH as solvent, wherein R is the same C₁₋₄ alkyl as in the 3-oxoalkanoate(II), the orthoester (III) and the alkali alkoxide (V).

As solvent in the halogenation step (iii), any solvent that is inert under the reaction conditions can be used. Preferred are aromatic hydrocarbons such as toluene, or halogenated hydrocarbons such as dichloromethane or chloroform.

The reaction temperatures are not critical, they are advantageously in the range of 60 to 100 °C for step (i), 0 to 50°C for step (ii), if a base is used, and 60 to 110°C for step (iii).

The reaction times depend on the reaction temperatures and the reactivities of the starting materials.

As mentioned above, the 2-hydroxypyrimidine-5-carboxylates may form hydrates such as those depicted in formula Ia above. Whether a hydrate is formed mainly depends on the nature of the substituents R¹ and R² and the work-up conditions. These hydrates may occur in several tautomeric forms and formula la represents the tautomer that is most consistent with the observed NMR data.

The 4-hydroxy-2-oxo-4-trifluoromethyl-1,2,3;4-tetrahydropyrimidine-5-carboxylates of formula Ia, wherein R is C₁₋₄ alkyl, R¹ is trifluoromethyl and R² is hydrogen or C₁₋₄ alkyl are novel compounds and also an object of the present invention. Particularly preferred are those 4-hydroxy-2-oxo-4-trifluoromethyl-1,2,3,4-tetrahydropyrimidine-5-carboxylates wherein R is methyl or ethyl and R² is hydrogen.

The following non-limiting examples will illustrate the invention.

### Example 1

### Ethyl 2-hydroxy-4-(trifluoromethyl)pyrimidine-5-carbozylate (I,R=Et,R¹=CF₃,R²=H,X=OH)

Under nitrogen atmosphere, urea (2.9 g, 0.05 mol), ethyl 4,4,4-trifluoro-3-oxobutyrate(8.9 g, 0.05 mol) and triethyl orthoformate (7.9g, 0.05 mol) were dissolved in ethanol (10 mL) and the solution was heated to 80 °C for 4 h. Then the reaction mixture was cooled to 20 °C and sodium ethoxide solution (21 wt.% in ethanol, 16.9 g, 0.05 mol) was added under stirring at the same temperature over 15 min. The reaction mixture was stirred for 2 h, followed by addition of water (75 mL) and acetic acid (2 mL) at 20 to 30°C. The resulting slurry was filtered and the filter cake was washed with water (20 mL) and dried at 45 °C.
Yield: 8.2 g (72%) white solid
¹H NMR (400 MHz, (CD₃)₂SO): δ = 8.69 (s, 1H), 4.24 (q, *J*= 7.1 Hz, 2H),1.28 (t, *J=* 7.1 Hz, 3H). ¹³C NMR (100 MHz, (CD₃)₂SO): δ =161.4, 158.7 (q, *J* = 35 Hz), 155.5, 154.7, 119.2 (q, *J*= 278 Hz), 105.7, 61.3, 13.7.

### Example 2

### Ethyl 2-hydroxy-4-(trifluoromethyl)pyrimidine-5-carboxylate Hydrate (Ethyl 4-hydroxy-2-oxo-4-trifluoromethyl-1,2,3,4-tetrahydropyrimidine-5-carboxylate-Ia, R = Et, R¹ = CF₃, R²=H)

Under nitrogen atmosphere, urea (1.63 kg, 27.1 mol), ethyl 4,4,4-trifluoro-3-oxobutyrate (5.00 kg, 27.1 mol) and triethyl orthoformate (4.43 kg, 29.9 mol) were dissolved in ethanol (5.0 L) and the solution was heated to 80 °C for 5 h. Then the reaction mixture was cooled to 20 °C and sodium ethoxide solution (21 wt.% in ethanol, 9.68 kg, 29.9 mol) was added under stirring at the same temperature over 1 to 2 h. The reaction mixture was stirred for another hour, followed by slow addition of a mixture of hydrochloric acid (33 wt.%, 4.50 kg, 40.7 mol) and water (15.0 L) at 20 to 30 °C. The resulting slurry was filtered and the filter cake was washed with water (24 L) and dried at 50 °C for 15 h.
Yield: 4.98 kg (72%) white solid
¹H NMR (400 MHz, (CD₃)₂SO): δ = 9.90 (d, *J*= 5.1 Hz, 1H, NH), 8.42 (s, 1H, NH), 7.49 (d, *J*= 6.1 Hz, 1H), 7.39 (s, 1H, OH), 4.16-4.06 (m, 2H), 1.20 (t, *J*= 7.0 Hz, 3H). ¹³C NMR (100 MHz, (CD₃)₂SO): δ = 163.6,149.4, 140.7, 123.5 (q, *J*= 290 Hz), 97.6, 81.6 (q, *J*= 34 Hz), 59.5, 14.0.

### Example 3

### Ethyl 2-chloro-4-(trifluoromethyl)pyrimidine-5-carboxylate (I,R=Et,R¹=CF₃,R²=H,X=Cl)

Ethyl 2-hydroxy-4-(trifluoromethyl)pyrimidine-5-carboxylate hydrate (3.00 kg, 11.8 mol; prepared according to Example 2) was dissolved in toluene (15 L) and *N*,*N*-dimethylformamide (0.46 kg, 6.4 mol) and thionyl chloride (3.78 kg, 31.8 mol) were added. The mixture was heated to 70 °C for 1 h under stirring and then cooled to room temperature. Water (9 L) was added and the mixture was warmed to 40 °C. The organic phase was separated and again treated with water (9 L) at 40 °C and separated. The solvent and the *N*,*N*-dimethylformamide were distilled off under reduced pressure (120→20 mbar, 40→60 °C) and the residue (3.4 kg) was distilled at 100 °C/5 mbar.
Yield: 2.66 kg (88%) colourless oil.
¹H NMR (400 MHz, CDCl₃): δ = 9.16 (s, 1H), 4.48 (q, *J*= 7.2 Hz, 2H), 1.44 (t, *J*= 7,2 Hz, 3H). ¹³C NMR (100 MHz, CDCl₃): δ = 163.1, 162.7, 162.3, 155.6 (q, *J*= 38 Hz), 123.2, 119.5 (q, *J*= 277 Hz), 63.5, 13.9.

### Example 4

### Methyl 2-hydroxy-4-(trifluoromethyl)pyrimidine-5-carboxylate (I,R=Me,R¹=CF₃,R²=H,X=OH)

Under nitrogen atmosphere, urea (14.1 g, 0.24 mol), methyl 4,4,4-trifluoro-3-oxobutyrate (40.0 g, 0.24 mol) and trimethyl orthoformate (27.5 g, 0.26 mol) were dissolved in methanol (35 mL) and the solution was heated to 65 °C for 20 h. Then the reaction mixture was cooled to 20°C and sodium methoxide (22.0 g, 0.41 mol) and methanol (25 mL) were added under stirring at the same temperature. The reaction mixture was stirred for 12 h, followed by addition of water (100 mL) and acetic acid (50 mL) at 20 to 30 °C. The resulting slurry was filtered and the filter cake was washed with water (80 mL) and dried at 50 °C.
Yield: 24.9 g (48%) white solid
¹H NMR (400 MHz, (CD₃)₂SO): δ =13.30 (br s, 1H, OH), 8.71 (s, 1H), 3.81 (s, 3H). ¹³C NMR (100 MHz, (CD₃)₂SO): δ = 161.8, 159.0 (q, *J*= 36 Hz), 155.6, 154.6, 119.2 (q, *J* = 278 Hz), 105.4, 52.4.

### Example 5

### Methyl 2-hydroxy-4-(trifluoromethyl)pyrimidine-5-carboxylate Hydrate (Methyl 4-hydroxy-2-oxo-4-trifluoromethyl-1,2,3,4-tetrahydropyrimidine-5-carboxylate Ia,R=Me,R¹=CF₃,R²=H)

Under nitrogen atmosphere, urea (12.0 g, 0.20 mol), methyl 4,4,4-trifluoro-3-oxobutyrate (33.5 g, 0.20 mol) and trimethyl orthoformate (23.3 g, 0.22 mol) were dissolved in methanol (34 mL) and the solution was heated to 65°C for 15 h. Then the reaction mixture was cooled to 20 °C and sodium methoxide solution (21 wt.% in methanol, 59.3 g, 0.23 mol) was added under stirring at the same temperature over 2 h. The reaction mixture was stirred for 20 h, followed by slow addition of a mixture of hydrochloric acid (37 wt.%, 21.7 g, 0.22 mol) and water (136 mL) at 20 to 30°C. The resulting slurry was filtered and the filter cake was washed with water (136 mL) and dried at 50 °C.
Yield: 17.5 g (37%) white solid
¹H NMR (400 MHz, (CD₃)₂SO): δ = 9.89 (d, *J*= 5.1 Hz, 1H, NH), 8.44 (s, 1H, NH), 7.50 (d, *J* = 6.0 Hz, 1 H), 7.42 (s, 1H, OH), 3.64 (s, 3H).
¹³C NMR (100 MHz, (CD₃)₂SO): δ = 164.0, 149.5, 141.0, 123.5 (q, *J*= 290 Hz), 97.4, 81.6 (q, *J*= 34 Hz), 51.1.

### Example 6

### Ethyl 2-hydroxy-4-methylpyrimidine-5-carboxylate (I,R=Et,R¹=Me,R²=H,X=OH)

Under nitrogen atmosphere, urea (15.7 g, 0.26 mol), ethyl 3-oxobutyrate (34.0 g, 0.26 mol) and triethyl orthoformate (42.6 g, 0.29 mol) were heated to 80 °C for 28 h while distilling off ethanol. Then the reaction mixture was cooled to 20 °C and ethanol (100 mL) and sodium ethoxide solution (21 wt.% in ethanol, 127.0 g, 0.39 mol) were added under stirring at the same temperature over 1 h. The reaction mixture was heated to 80 °C and stirred for 2 h. Thereafter, the reaction mixture was cooled to 20 °C, followed by addition of water (136 mL) and acetic acid (19 mL) at 20 to 30 °C. The resulting slurry was filtered and the filter cake was washed with water (172 mL) and dried at 50 °C.
Yield: 23.1 g (48%) off-white solid
¹H NMR (400 MHz, (CD₃)₂SO): δ = 8.72 (s, 1H), 4.22 (q, *J*= 7.1 Hz, 2H), 2.50 (s, 3H), 1.28 (t, *J* = 7.1 Hz, 3H).
¹³C NMR (100 MHz, (CD₃)₂SO): δ = 167.3, 163.6, 162.1, 156.6, 105.9, 60.1, 21.0, 14.0.

### Example 7

### Ethyl 2-hydroxy-4-phenylpyrimidine-5-carboxylate (I,R=Et,R¹=Me,R²=H,X=OH)

Under nitrogen atmosphere, urea (7.8 g, 0.13 mol), ethyl benzoylacetate(25.0 g, 0.13 mol) and triethyl orthoformate (23.3 g, 0.16 mol) were heated to 80 °C for 20 h while distilling off ethanol. Then the reaction mixture was cooled to 20 °C and ethanol (150 mL) and sodium ethoxide solution (21 wt.% in ethanol, 63.2 g, 0.20 mol) were added under stirring at the same temperature over 1 h. The reaction mixture was heated to 80 °C and stirred for 2 h. Thereafter, the reaction mixture was cooled to 20 °C, followed by addition of water (80 mL) and acetic acid (20 mL) at 20 to 30 °C. The resulting solution was extracted with ethyl acetate (2×150 mL) The combined organic phases were evaporated and purified by chromatography on SiO₂.
Yield: 6.3 g (27%) off-white solid
¹H NMR (400 MHz, (CD₃)₂SO): δ = 12.50 (br s, 1H, OH), 8.61 (s, 1H), 7.52-7.42 (m, 5H), 4.03 (q, *J*= 7.1 Hz, 2H), 1.01 (t, *J*= 7.1 Hz, 3H).
¹³C NMR (100 MHz, (CD₃)₂SO): δ = 163.7, 155.2, 130.0, 128.1, 127.7, 107.3, 60.4, 13.5.

### Example 8

### Methyl 2-hydroxy-4-(trifluoromethyl)pyrimidine-5-carboxylate Hydrate (Methyl 4-hydroxy-2-oxo-4-trifluoromethyl-1,2,3,4-tetrahydropyrimidine 3-carboxylate - Ia,R=Me,R¹=CF₃,R²=H)

Under nitrogen atmosphere, urea (8.8 g, 0.15 mol), methyl 4,4,4-trifluoro-3-oxobutyrate (25.0 g, 0.15 mol) and trimethyl orthoformate (17.2 g, 0.16 mol) were dissolved in methanol (25 mL) and the solution was heated to 65 °C for 5 h. Then the reaction mixture was cooled to 0 °C. The resulting slurry was filtered and the filter cake was washed with methanol (100 mL) and dried at 50 °C.
Yield: 12.8 g(3,6%) white solid
NMR: See Example 5.

### Example 9

### Ethyl 2-hydroxy-4-(trifluoromethyl)pyrimidine-5-carboxylate Hydrate (Ethyl 4-hydroxy-2-oxo-4-trifluoromethyl-1,2,3,4-tetrahydropyrimidine-5-carboxylate-Ia,R=Et,R¹=CF₃,R²=H)

Under nitrogen atmosphere, urea (13.1 kg, 217.0 mol), ethyl 4,4,4-trifluoro-3-oxobutyrate (39.9 kg, 216.3 mol) and triethyl orthoformate (35.4 kg, 239.1 mol) were dissolved in ethanol (40 L) and the solution was heated to 80 °C for 5 h. Then the reaction mixture was cooled to 0 °C. The resulting slurry was filtered and the filter cake was washed with ethanol (40 L) and dried at 50 °C for 15h.
Yield: 40.1 kg (73%) white solid
NMR: See Example 2.

### Example 10

### Ethyl 2-chloro-4-(trifluoromethyl)pyrimidine-5-carboxylate (I,R=Et,R¹=CF₃,R²=H,X=Cl)

Ethyl 2-hydroxy-4-(trifluoromethyl)pyrimidine-5-carboxylate (2.0 g, 0.01 mol) was dissolved in toluene (20 mL) and *N,N*-dimethylformamide (0.31 g, ca. 0.004 mol) and thionyl chloride (5.05 g, 0.04 mol) were added. The mixture was heated to 70 °C for 2 h under stirring and then cooled to room temperature. The solution was washed with water (3×20 mL). The solvent and the *N,N*-dimethylformamide were distilled offunder reduced pressure (120→30 mbar, 45 °C) to afford the crude product as a yellow oil (2.3 g).

## Claims

1. A process for the production of pyrimidine-5-carboxylates of formula 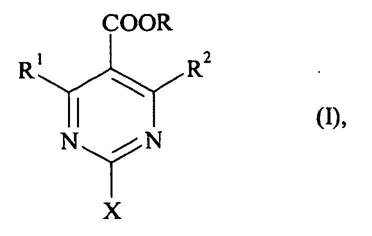 wherein
R is C₁₋₄ alkyl,
R¹ is C₁₋₄ alkyl, trifluoromethyl or optionally substituted phenyl,
R² is hydrogen or C₁₋₄ alkyl
and X is hydroxy, chlorine or bromine;
or, if X is hydroxy, a hydrate thereof,
said process comprising the steps of
(i) reacting a 3-oxoalkanoate of formula 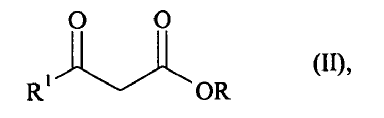 wherein R and R¹ are as defined above, with urea and an orthoester of formula
R²C(OR)₃ (III),
wherein R and R² are as defined above, to yield a 2-acyl-3-ureidoacrylate of formula 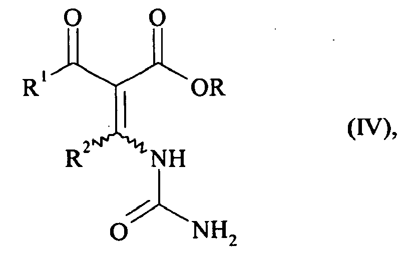 wherein R, R¹ and R² are as defined above,
(ii) reacting said 2-acyl-3-ureidoacrylate (IV) to give a 2-hydroxypyrimidine-5-carboxylate of formula 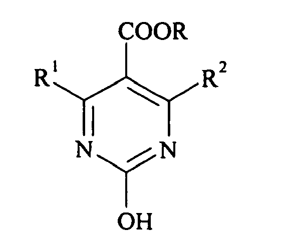 (I), X = OH
wherein R, R¹ and R² are as defined above, or a hydrate thereof, and, optionally,
(iii) converting said 2-hydroxypyrimidine-5-carboxylate or hydrate thereof into a corresponding chloro or bromo compound (I, X = Cl, Br), **characterised in that** steps (i) and (ii) are conducted in a one-pot reaction without isolating any intermediate.

2. The process of claim 1, wherein step (ii) is carried out in the presence of an alkali alkoxide of formula
M-OR (V),
wherein M is an alkali metal and R is as defined above.

3. The process of claim 1, wherein step (ii) is carried out without addition of a base.

4. The process of any of claims 1 to 3, wherein R is ethyl.

5. The process of any of claims 1 to 4, wherein R¹ is trifluoromethyl.

6. The process of any of claims 1 to 5, wherein R² is hydrogen.

7. The process of any of claims 1 to 6, wherein X is chlorine.

8. The process of claim 7, wherein the conversion in step (iii) is conducted with phosphorus oxychloride or thionyl chloride.

9. The process of claim 8, wherein the conversion in step (iii) is conducted with thionyl chloride in the presence of *N,N*-dimethylformamide.

10. The process of any of claims 1 to 9 wherein steps (i) and (ii) are conducted in an alcohol of formula R-OH as solvent, wherein R is as defined in claim 1.

## Patentansprüche

1. Verfahren zur Herstellung von Pyrimidin-5-carboxylaten der Formel 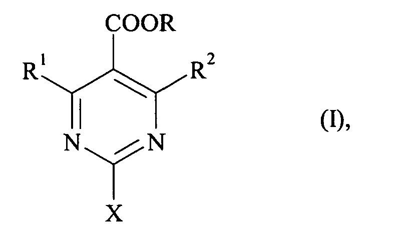 worin
R C₁₋₄-Alkyl,
R¹ C₁₋₄-Alkyl, Trifluormethyl oder gegebenenfalls substituiertes Phenyl,
R² Wasserstoff oder C₁₋₄-Alkyl
und X Hydroxy, Chlor oder Brom bedeuten,
oder, falls X Hydroxy ist, deren Hydraten,
umfassend die Schritte:
(i) Umsetzung eines 3-Oxoalkanoats der Formel 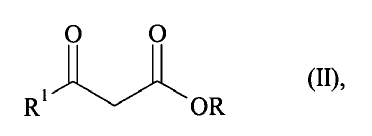 worin R und R¹ wie oben definiert sind, mit Harnstoff und einem Orthoester der Formel
R²C(OR)₃ (III),
worin R and R² wie oben definiert sind, zu einem 2-Acyl-3-ureidoacrylat der Formel 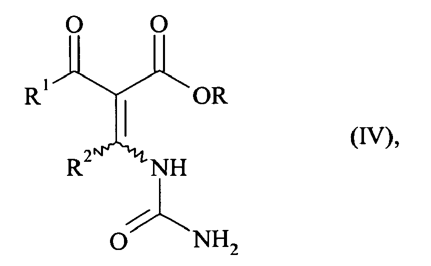 worin R, R¹ and R² wie oben definiert sind,
(ii) Umsetzung des 2-Acyl-3-ureidoacrylats (IV) zu einem 2-Hydroxypyrimidin-5-carboxylat der Formel 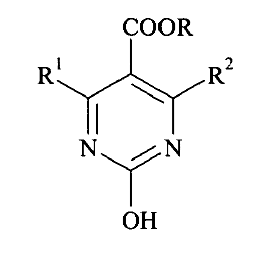 (I), X = OH
worin R, R¹ and R² wie oben definiert sind, oder dessen Hydrat, und gegebenenfalls
(iii) Überführung des 2-Hydroxypyrimidin-5-carboxylats oder dessen Hydrats in eine entsprechende Chlor- oder Bromverbindung (I, X = Cl, Br),
**dadurch gekennzeichnet, dass** die Schritte (i) und (ii) in einer Eintopfreaktion ohne Isolierung eines Zwischenprodukts durchgeführt werden.

2. Verfahren nach Anspruch 1, worin Schritt (ii) in Gegenwart eines Alkalialkoxids der Formel
M-OR (V),
worin M ein Alkalimetall und R wie oben definiert ist, durchgeführt wird.

3. Verfahren nach Anspruch 1, worin Schritt (ii) ohne Zusatz einer Base durchgeführt wird.

4. Verfahren nach einem der Ansprüche 1 bis 3, worin R Ethyl ist.

5. Verfahren nach einem der Ansprüche 1 bis 4, worin R¹ Trifluormethyl ist.

6. Verfahren nach einem der Ansprüche 1 bis 5, worin R² Wasserstoff ist.

7. Verfahren nach einem der Ansprüche 1 bis 6, worin X Chlor ist.

8. Verfahren nach Anspruch 7, worin die Umsetzung in Schritt (iii) mit Phosphoroxychlorid oder Thionylchlorid durchgeführt wird.

9. Verfahren nach Anspruch 8, worin die Umsetzung in Schritt (iii) mit Thionylchlorid in Gegenwart von *N,N*-Dimethylformamid durchgeführt wird.

10. Verfahren nach einem der Ansprüche 1 bis 9, worin die Schritte (i) und (ii) in einem Alkohol der Formel R-OH als Lösungsmittel durchgeführt werden, wobei R wie in Anspruch 1 definiert ist.

## Revendications

1. Procédé de production de pyrimidine-5-carboxylates de formule : 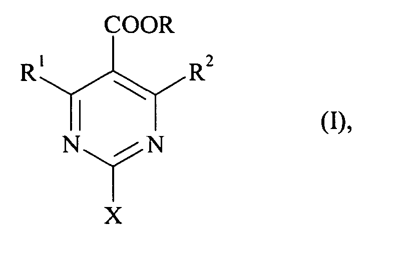 dans laquelle
R est un groupe alkyle en C₁₋₄,
R¹ est un groupe alkyle en C₁₋₄, un groupe trifluorométhyle ou un groupe phényle éventuellement substitué,
R² est un atome d'hydrogène ou un groupe alkyle en C₁₋₄,
et X est un groupe hydroxy, un atome de chlore ou un atome de brome ;
ou, si X est un groupe hydroxy, d'un hydrate de celui-ci,
ledit procédé comprenant les étapes consistant à :
(i) faire réagir un 3-oxoalcanoate de formule : 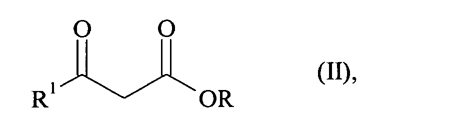 dans laquelle R et R¹ sont tels que définis ci-dessus, avec de l'urée et un ortho-ester de formule :
R²C(OR)₃ (III),
dans laquelle R et R² sont tels que définis ci-dessus, pour donner lieu à un 2-acyl-3-uréidoacrylate de formule : 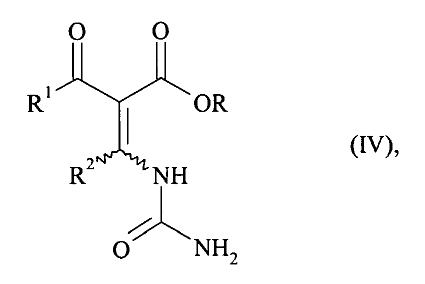 dans laquelle R, R¹ et R² sont tels que définis ci-dessus,
(ii) faire réagir ledit 2-acyl-3-uréidoacrylate (IV) pour donner lieu à un 2-hydroxypyrimidine-5-carboxylate de formule : 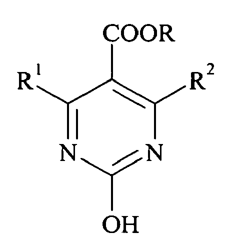 (I), X = OH
dans laquelle R, R¹ et R² sont tels que définis ci-dessus, ou à un hydrate de celui-ci, et, éventuellement,
(iii) transformer ledit 2-hydroxypyrimidine-5-carboxylate ou hydrate de celui-ci en un composé chloro ou bromo correspondant (I, X = Cl, Br), **caractérisé en ce que** les étapes (i) et (ii) sont réalisées dans une réaction en un seul récipient sans isoler aucun intermédiaire.

2. Procédé selon la revendication 1, dans lequel l'étape (ii) est réalisée en présence d'un alcoxyde de métal alcalin de formule :
M-OR (V),
dans laquelle M est un métal alcalin et R est tel que défini ci-dessus.

3. Procédé selon la revendication 1, dans lequel l'étape (ii) est réalisée sans l'addition d'une base.

4. Procédé selon l'une quelconque des revendications 1 à 3, dans lequel R est un groupe éthyle.

5. Procédé selon l'une quelconque des revendications 1 à 4, dans lequel R¹ est un groupe trifluorométhyle.

6. Procédé selon l'une quelconque des revendications 1 à 5, dans lequel R² est un atome d'hydrogène.

7. Procédé selon l'une quelconque des revendications 1 à 6, dans lequel X est un atome de chlore.

8. Procédé selon la revendication 7, dans lequel la transformation dans l'étape (iii) est réalisée avec de l'oxychlorure de phosphore ou du chlorure de thionyle.

9. Procédé selon la revendication 8, dans lequel la transformation dans l'étape (iii) est réalisée avec du chlorure de thionyle en présence de *N,N*-diméthylformamide.

10. Procédé selon l'une quelconque des revendications 1 à 9, dans lequel les étapes (i) et (ii) sont réalisées dans un alcool de formule R-OH en tant que solvant, dans lequel R est tel que défini dans la revendication 1.
